# EUROPEAN PATENT APPLICATION

(11) **EP 3 792 273 A1**
(43) Date of publication of application: **17.03.2021**
(21) Application number: 20802069.3
(22) Date of filing: 06.05.2020
(51) Int. Cl.: C07K 7/16, C07K 1/20

(54) **REFINING METHOD FOR VASOPRESSIN**

(30) Priority: 06.05.2019 CN 201910371792
(71) Applicant: SPH No.1 Biochemical & Pharmaceutical Co. Ltd., Shanghai 200240 (CN)
(72) Inventor: JIANG, Ximing, Shanghai 200240 (CN); ZHU, Xinlei, Shanghai 200240 (CN); HUANG, Long, Shanghai 200240 (CN); DING, Jinguo, Shanghai 200240 (CN); HUANG, Zhenhui, Shanghai 200240 (CN)
(74) Representative: Vitina, Maruta
(86) International application number: PCT/CN2020/088716
(87) International publication number: WO 2020/224580

(57) **Abstract**

A method for refining vasopressin, including: subjecting a crude vasopressin solution to reversed-phase enrichment, reversed-phase salt conversion and reversed-phase purification sequentially using reversed-phase high performance liquid chromatography. The crude vasopressin solution is obtained by oxidizing a crude reduced vasopressin prepared by solid phase synthesis. A super water-resistant packing material is used in the reversed-phase high performance liquid chromatography. This method adopts a combination of on-line reversed-phase enrichment, reversed-phase salt conversion and reversed-phase purification to produce pure polypeptide, which allows for high removal rate of impurities, high purity and optimized operation. Moreover, the mobile phases used in the process of column equilibration, sample loading and salt conversion are all aqueous solutions, which can be directly treated and recycled after use.

## Description

### TECHNICAL FIELD

This application relates to peptide purification, and more particularly to a method for refining vasopressin.

### BACKGROUND

Vasopressin is a synthetic peptide composed of nine amino acid residues and has a theoretical molecular weight of 1084.24, as shown in the following formula: Cys-Tyr-Phe-Gln-Asn-Cys-Pro-Arg-Gly-NH2 . Vasopressin, also named angiotensin and pitressin, pertains to neurohypophysial hormones and has two kinds of receptors V1 and V2. V1 is mainly distributed on the membrane of vascular smooth muscle cells, and plays a role in constricting blood vessels and raising blood pressure via a receptor-G protein-second messenger pathway. V2 is distributed on the epithelial cells of the distal tubules and collecting ducts of the kidney, and a physiological dose of V2 can promote the reabsorption of water at the renal distal tubules and collecting ducts to exert an antidiuretic effect.

At present, most of commercially-available peptide drugs are purified through preparative high performance liquid chromatography (HPLC), which is considered to be the most effective tool for obtaining high-purity target peptide molecules. Generally, in the preparation of peptide drugs, the target peptide is first enriched by medium and low pressure chromatography and then refined by high pressure chromatography. However, there is no suitable molecular sieve gel column (small loading amount, low flow rate and small processing capacity; suitable for the desalting of proteins with a molecular weight greater than 10 kDa) or ultrafiltration membrane for the purification of vasopressin since its molecular weight is extremely low (about 1 kDa). Moreover, the commonly-used medium-low pressure chromatography methods, such as molecular sieve chromatography, ion exchange chromatography and hydrophobic interaction chromatography, generally adopt a packing material with a particle size of tens of microns to hundreds of microns and a pore size of hundreds of nanometers, and thus they cannot be applied to the preparation of high-purity vasopressin. The crude vasopressin product prepared through the combination of solid phase synthesis and high-dilution cyclization has a relatively low concentration, and when the crude product is purified by ordinary reversed-phase chromatography, a considerable amount of organic waste will be generated during the loading process, increasing the disposal cost of hazardous waste. Currently, since there is still a lack of an effective method for preparing peptide salt APIs (active pharmaceutical ingredients), it is urgent to develop a new economical and effective process for purifying low-concentration peptides and their salts.

Chinese patent application publication No. 106518975A discloses a method for preparing vasopressin, in which a crude vasopressin precursor solution is sequentially subjected to reversed-phase cyclization, reversed-phase purification and reversed-phase desalting using reversed-phase HPLC; the packing material used in the reversed-phase HPLC is a styrene-divinylbenzene copolymer; and the vasopressin precursor carries two free sulfhydryl groups and is used as starting material. However, this method fails to effectively remove impurities in the crude product (removal rate: 14.3%), and meanwhile, the mobile phase contains NaOH as an alkali, which is unfavorable for pH control and may affect the stability of the target product. In addition, the cyclization needs a large amount of organic solvent as mobile phase, and the subsequent purification and salt conversion will generate a large amount of hazardous organic waste, bringing an increase in the disposal cost and the difficulty in recycling.

### SUMMARY

An object of the disclosure is to provide a method for refining vasopressin to overcome the defects in the prior art, such as unsatisfactory removal rate of impurities, large consumption of organic solvents and high cost for treating organic hazardous waste. The method provided herein is economical and environmentally friendly since most of the generated waste liquid can be directly reused through sewage treatment. Moreover, the method also has a high removal rate of impurities and a desired purity.

The technical solutions of the disclosure are described as follows.

The disclosure provides a method for refining vasopressin, comprising:
subjecting a crude vasopressin solution to reversed-phase enrichment, reversed-phase salt conversion and reversed-phase purification sequentially using a reversed-phase high performance liquid chromatography (RP-HPLC);
wherein a packing material used in the PR-HPLC is a water-resistant packing material;
the reversed-phase enrichment, the reversed-phase salt conversion and the reversed-phase purification are all completed in one reversed-phase elution, and conditions of the reversed-phase elution are listed as follows:

| Steps | Time | Eluent |
|---|---|---|
| 1 | 0-50 min | 100% sample C1 |
| 2 | 51-71 min | 100% mobile phase C2 |
| 3 | 72-90 min | 100% mobile phase A |
| 4 | 90-95 min | 100% mobile phase A→90% mobile phase A+10% mobile phase B |
| 5 | 95-125 min | 90% mobile phase A+10% mobile phase B→80% mobile phase A+20% mobile phase B |

wherein the mobile phase A consists of 0.005-0.1% by volume of acetic acid and water; the mobile phase B consists of 0.005-0.1% by volume of acetic acid and acetonitrile; the sample C1 is the crude vasopressin solution; the mobile phase C2 is a 5-50 mM aqueous NH₄Ac-NH₄OH solution at pH 7.0-9.0; and a flow rate of the eluent is 80-100 mL/min;
collecting an eluate within a retention time of 105-115 min to obtain a pure vasopressin solution.

In an embodiment, the crude vasopressin solution is obtained by dissolving, diluting, and oxidizing a crude reduced vasopressin product prepared by solid phase synthesis.

In an embodiment, the crude vasopressin solution is prepared as follows. Rink Amide MBHA resin is used as solid support, and Fmoc-protected amino acids are coupled one by one in the presence of HOBt/DIC (condensing agent) to form a peptide. The peptide is cleaved from the resin under the action of a cleaving agent and precipitated with methyl tert-butyl ether to obtain crude reduced vasopressin. Then the crude reduced vasopressin is dissolved with a 50% aqueous acetic acid solution and diluted with water to obtain the crude reduced vasopressin solution, which is subsequently adjusted to pH 7.0-9.0 with a basic substance and added with 30% hydrogen peroxide (0.5 mL per gram of crude reduced vasopressin) for oxidation to obtain a crude oxidized vasopressin solution, that is, the crude vasopressin solution.

In some embodiments, the 50% aqueous acetic acid solution can completely dissolve the crude reduced vasopressin.

In some embodiments, a concentration of the crude reduced vasopressin solution is 0.1-4 mg/mL, preferably 0.5-2 mg/mL.

In some embodiments, the cleaving agent is a conventional cleaving agent in the art, preferably a mixture of trifluoroacetic acid, triisopropylsilane and water in a volume ratio of 90: 7.5: 2.5 (TFA: TIS: H₂O = 90: 7.5: 2.5).

In some embodiments, the basic substance is NaOH.

In an embodiment, a formula of the vasopressin in the crude vasopressin solution is Cys-Tyr-Phe-Gln-Asn-Cys-Pro-Arg-Gly-NH2 ; and a solvent of the crude vasopressin solution is an aqueous solution containing trifluoroacetic acid and acetic acid.

In some embodiments, the water-resistant packing material is UniSil® ODS-AQ material.

In some embodiments, the water-resistant packing material has a pore size of 7-10 nm and a particle size of 10 µm.

In an embodiment, the packing material is UniSil® ODS-AQ material with a pore size of 10 nm and a particle size of 10 µm, and the packing is performed by a Load&Lock dynamic axial compression and static locking technology at a pressure of 1000 psi using a Varian chromatography column packing station. Specifically, 300 g of powdered UniSil® ODS-AQ material is mixed evenly with 600 mL of isopropanol under stirring, and then the mixture is poured into a Load&Lock4002 preparation column with an inner diameter of 50 mm, where the compression ratio is 1.5: 1; the carrier gas is N₂, which is adjusted such that a pressure displayed on a oil pressure gauge is 1500 psi; and the dynamic axial compression is performed to enable that the column bed length is 25 cm. The obtained preparative column is used in the reversed-phase enrichment, reversed-phase salt conversion and reversed-phase purification.

In some embodiments, a detection wavelength of the RP-HPLC is 220 nm.

In some embodiments, the mobile phase A consists of 0.02-0.05% by volume of acetic acid and water; and/or
the mobile phase B consists of 0.02-0.05% by volume of acetic acid and acetonitrile; and/or
the mobile phase C2 is a 10-20 mM aqueous NH₄Ac-NH₄OH solution; and/or
the pH of the mobile phase C2 is 7.5-8.5; and/or
a HPLC purity of the crude vasopressin in the crude vasopressin solution is 60-85%, preferably 70%-85%, and more preferably 70%-80%.

In some embodiments, during an elution period of 50-51 min, the eluent is changed from sample C1 to mobile phase C2; during an elution period of 71-72 min, the eluent is changed from mobile phase C2 to mobile phase A. It should be understood by those skilled in the art that the above period is not intended to limit the elution conditions of the disclosure, and this period can be adjusted according to the model of the HPLC system.

In some embodiments, during a period of 125-126 min after step (5) of the elution, a proportion of mobile phase A in the eluent decreases uniformly from 80% to 50%, and a proportion of the mobile phase B in the eluent uniformly increases to 50% correspondingly; and during the period of 126-135 min, the eluent is kept constant in the composition (50% mobile phase A and 50% mobile phase B) to clean the chromatographic column.

In some embodiments, the reversed-phase enrichment corresponds to the elution step (1), and the steps (2) and (3) correspond to the reversed-phase salt conversion. Specifically, in step (2), a weak base NH₄Ac-NH₄OH is introduced to remove trifluoroacetate in the crude vasopressin; the step (3) is the process of removing ammonium ion introduced in step (2). The reversed-phase purification is performed in steps (4) and (5), and in step (4), the impurities with weak adsorption are removed.

In some embodiments, in step (4) of the elution, the proportion of the mobile phase B in the eluent increases by 2% per minute, and the proportion of mobile phase A decreases by 2% accordingly. In step (5) of the elution, the proportion of the mobile phase B in the eluent increases by 0.333% per minute, and the proportion of the mobile phase A decreases by 0.333% accordingly.

The vasopressin is a polypeptide, which is unstable and prone to degradation under high pH, especially in an alkaline environment. The disclosure comprehensively investigates the pH and time of salt conversion to minimize the damage and loss of the target product in the salt conversion process.

The above conditions can be arbitrarily combined to obtain preferred embodiments of the invention.

Unless otherwise specified, the reagents and raw materials used herein are commercially available.

Compared to the prior art, the disclosure has the following beneficial effects.
(1) Through the use of a packing material with high water resistance and adsorption performance, the target peptide is absorbed by the stationary phase through hydrophobic interaction to achieve the online enrichment. Then the composition of the eluent is adjusted to perform gradient elution and purification to obtain the final pure product. This method is suitable for the continuous production of high-purity peptides.
(2) The invention innovatively optimizes the production process through the combination of reversed-phase adsorption enrichment, salt conversion and desalting, and this method is promising in the industrial application. Moreover, the method provided herein has higher removal rate of impurities and purity of the final product.
(3) A new application of the super water-resistant packing material is designed. The eluents used in the process of column equilibration, reversed-phase enrichment and reversed-phase salt conversion are all aqueous solutions, which can be directly treated and recycled after use. Compared to the traditional preparation process, the method of the disclosure greatly reduces the generation of hazardous waste liquid, and thus is suitable for the economic and green production.

### DETAILED DESCRIPTION OF EMBODIMENTS

The invention will be further described below in detail with reference to the embodiments, but the invention is not limited to these embodiments. Unless otherwise specified, the experiments in the following embodiments are performed according to conventional methods and conditions, or according to the instruction of the manufacturer.

The UniSil® ODS-AQ super water-resistant packing material (pore size: 10 nm; particle size: 10 µm) used in the embodiments is purchased from Suzhou NanoMicro Technology Co., Ltd.

The purity of the crude and purified vasopressin solutions is detected by HPLC, where the HPLC parameters are listed as follows.
Instrument: Agilent 1260 High Performance Liquid Chromatograph;
Chromatographic column: Waters XBridge C18 (4.6×250 mm, 5 µm);
Mobile phase: A: an aqueous solution containing 0.1% by volume of trifluoroacetic acid; B: an aqueous solution containing 0.1% by volume of trifluoroacetic acid and 50% by volume of acetonitrile;
Flow rate: 1.0 mL/min;
Detection wavelength: 210 nm;
Column temperature: 25°C.

The elution gradient is shown in Table 1, where the percentage is calculated by volume.

**Table 1 Elution program for HPLC determination of vasopressin**

| Step | Time | Eluent |
|---|---|---|
| 1 | 0-2 min | 95% A+5% B |
| 2 | 2-12 min | 95%A+5% B→85%A+15% B |
| 3 | 12-22 min | 85% A+15% B |
| 4 | 22-30 min | 85% A+15% B→77%A+23% B |
| 5 | 30-30.1 min | 77% A+23% B→ 50% A+50% B |
| 6 | 30.1-35 min | 50% A+50% B |

The crude vasopressin solution is obtained by dissolving, diluting, and oxidizing a crude reduced vasopressin synthesized by solid phase synthesis. Specifically, Rink Amide MBHA resin is used as solid support, and Fmoc-protected amino acids are coupled one by one in the presence of HOBt/DIC (condensing agent) to form a peptide. The peptide is cleaved from the resin under the action of a cleaving agent and precipitated with methyl tert-butyl ether to obtain crude reduced vasopressin, where the cleaving agent is a mixture of TFA, TIS and H₂O in a volume ratio of 90: 7.5: 2.5. Then the crude reduced vasopressin is dissolved with a 50% aqueous acetic acid solution, and diluted with water to obtain the crude reduced vasopressin solution, which is subsequently adjusted to pH 7.0-9.0 with a basic substance and added with 30% hydrogen peroxide (0.5 mL per gram of crude reduced vasopressin) for oxidation to obtain a crude oxidized vasopressin solution, where the basic substance is NaOH.

### Example 1 Packing of L&L4002 preparation column with an inner diameter of 50 mm

UniSil® ODS-AQ material with a pore size of 10 nm and a particle size of 10 µm was used as packing material, and the packing was performed by a Load&Lock dynamic axial compression and static locking technology at a pressure of 1000 psi using a Varian chromatography column packing station. Specifically, 300 g of powdered UniSil® ODS-AQ material was mixed evenly with 600 mL of isopropanol under stirring, and then the mixture was poured into the Load&Lock4002 preparation column with an inner diameter of 50 mm, where the compression ratio was 1.5: 1; the carrier gas was N₂, which was adjusted such that a pressure displayed on a oil pressure gauge was 1500 psi; and the dynamic axial compression is performed to enable that the column bed length was 25 cm. The obtained preparation column was used in the reversed-phase enrichment, salt conversion and purification.

### Example 2 Reversed-phase enrichment, reversed-phase salt conversion and reversed-phase purification of crude vasopressin solution

Instrument: Varian SD-1 preparative high-pressure liquid chromatograph system.

Column: self-prepared preparation column Load&Lock4002 (50×250 mm, UniSil® ODS-AQ (particle size: 10 µm; pore size: 10 nm)).

The vasopressin had a structural formula of 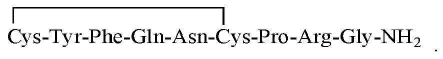
Cys-Tyr-Phe-Gln-Asn-Cys-Pro-Arg-Gly-NH2 .
 The concentration of the crude reduced vasopressin in the crude reduced vasopressin solution was 0.1 mg/mL, and the solvent of the crude vasopressin solution was an aqueous solution containing trifluoroacetic acid and acetic acid.

The mobile phase A was a 0.02% aqueous acetic acid solution; the mobile phase B consisted of 0.02% by volume of acetic acid and acetonitrile; the sample C1 was the crude vasopressin solution, in which the vasopressin had a HPLC purity of 73.63%; and the mobile phase C2 was a 10 mM aqueous NH₄Ac-NH₄OH solution (pH 7.5).

The conditions for reversed-phase enrichment, reversed-phase salt conversion and reversed-phase purification were listed as follows: flow rate: 1.0 mL/min; detection wavelength: 220 nm; and elution gradient was shown in Table 2 ("%" referred to percentage by volume).

**Table 2 Elution program for reversed-phase enrichment, salt conversion and purification**

| Step | Time | Eluent |
|---|---|---|
| 1 | 0-50 min | 100% sample C1 |
| 2 | 51-71 min | 100% mobile phase C2 |
| 3 | 72-90 min | 100% mobile phase A |
| 4 | 90-95 min | 100% mobile phase A→90% mobile phase A+10% mobile phase B |
| 5 | 95-125 min | 90% mobile phase A+10% mobile phase B→80% mobile phase A+20% mobile phase B |

During an elution period of 125-126 min after the step (5), a proportion of mobile phase A in the eluent uniformly decreased from 80% to 50%, and a proportion of the mobile phase B in the eluent uniformly increased to 50% correspondingly. During the period of 126-135 min, the eluent was kept constant in the composition (50% mobile phase A and 50% mobile phase B) to clean the chromatographic column. The eluate with a retention time of 105-115 min was collected as the purified vasopressin solution, which was measured by HPLC to have a vasopressin purity of 99.56%.

The removal rate of impurities in the crude vasopressin solution was 25.93%. The eluents used in the steps (1) to (3) were all aqueous solutions, which can be directly treated and recycled after use and will not pollute the environment. Compared with the traditional preparation process, the method provided herein greatly reduced the generation of hazardous waste liquid, lowering the treatment cost and avoiding environmental pollution.

### Example 3 Reversed-phase enrichment, reversed-phase salt conversion and reversed-phase purification of crude vasopressin solution

Instrument: Varian SD-1 preparative high-pressure liquid chromatograph system.

Column: self-prepared preparation column Load&Lock4002 (50×250 mm, UniSil® ODS-AQ (particle size: 10 µm; pore size: 10 nm)).

The vasopressin had a structural formula of 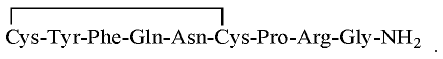
Cys-Tyr-Phe-Gln-Asn-Cys-Pro-Arg-Gly-NH2 .
 The concentration of the crude reduced vasopressin in the crude reduced vasopressin solution was 1.5 mg/mL, and the solvent of the crude vasopressin solution was an aqueous solution containing trifluoroacetic acid and acetic acid.

The mobile phase A was a 0.05% aqueous acetic acid solution; the mobile phase B consisted of 0.05% by volume of acetic acid and acetonitrile; the sample C1 was the crude vasopressin solution, in which the vasopressin had a HPLC purity of 75.23%; and the mobile phase C2 was a 20 mM aqueous NH₄Ac-NH₄OH solution (pH 8.5).

The conditions for reversed-phase enrichment, reversed-phase salt conversion and reversed-phase purification were listed as follows: flow rate: 100 mL/min; detection wavelength: 220 nm; and elution gradient was shown in Table 3 ("%" referred to percentage by volume).

**Table 3 Elution program for reversed-phase enrichment, salt conversion and purification**

| Steps | Time | Eluent |
|---|---|---|
| 1 | 0-50 min | 100% sample C1 |
| 2 | 51-71 min | 100% mobile phase C2 |
| 3 | 72-90 min | 100% mobile phase A |
| 4 | 90-95 min | 100% mobile phase A→90% mobile phase A+10% mobile phase B |
| 5 | 95-125 min | 90% mobile phase A+10% mobile phase B→80% mobile phase A+20% mobile phase B |

During an elution period of 125-126 min after the step (5), a proportion of the mobile phase A in the eluent uniformly decreased from 80% to 50% mobile phase A, and a proportion of the mobile phase B in the eluent uniformly increased to 50% correspondingly. During the period of 126-135 min, the eluent was kept constant in the composition (50% mobile phase A and 50% mobile phase B) to clean the chromatographic column. The eluate with a retention time of 105-115 min was collected as the purified vasopressin solution, which was measured by HPLC to have a vasopressin purity of 99.62%. In this example, the removal rate of impurities in the crude vasopressin solution reached 24.39%.

### Example 4 Reversed-phase enrichment, reversed-phase salt conversion and reversed-phase purification of crude vasopressin solution

Instrument: Varian SD-1 preparative high-pressure liquid chromatograph system

Column: self-prepared preparation column Load&Lock4002 (50×250 mm, UniSil® ODS-AQ (particle size: 10 µm; pore size: 10 nm)).

The vasopressin had a structural formula of Cys-Tyr-Phe-Gln-Asn-Cys-Pro-Arg-Gly-NH2 . The concentration of the crude reduced vasopressin in the crude reduced vasopressin solution was 0.8 mg/mL, and the solvent of the crude vasopressin solution was an aqueous solution containing trifluoroacetic acid and acetic acid.

The mobile phase A was a 0.05% aqueous acetic acid solution; the mobile phase B consisted of 0.05% by volume of acetic acid and acetonitrile; the sample C1 was the crude vasopressin solution, in which the vasopressin had a HPLC purity of 75.66%; and the mobile phase C2 was a 20 mM aqueous NH₄Ac-NH₄OH solution (pH 7.5).

The conditions for reversed-phase enrichment, reversed-phase salt conversion and reversed-phase purification were listed as follows: flow rate: 100 mL/min; detection wavelength: 220 nm; and elution gradient was shown in the Table 4 ("%" referred to percentage by volume).

**Table 4 Elution program for reversed-phase enrichment, salt conversion and purification**

| Steps | Time | Eluent |
|---|---|---|
| 1 | 0-50 min | 100% sample C1 |
| 2 | 51-71 min | 100% mobile phase C2 |
| 3 | 72-90 min | 100% mobile phase A |
| 4 | 90-95 min | 100% mobile phase A→90% mobile phase A+10% mobile phase B |
| 5 | 95-125 min | 90% mobile phase A+10% mobile phase B→80% mobile phase A+20% mobile phase B |

During an elution period of 125-126 min after the step (5), a proportion of the mobile phase A in the eluent uniformly decreased from 80% mobile phase A to 50%, and a proportion of the mobile phase B in the eluent uniformly increased to 50% correspondingly. During the period of 126-135 min, the eluent was kept constant in the composition (50% mobile phase A and 50% mobile phase B) to clean the chromatographic column. The eluate with a retention time of 105-115 min was collected as the purified vasopressin solution, which was measured by HPLC to have a vasopressin purity of 99.52%. The removal rate of impurities in the crude vasopressin solution was 23.86%.

### Example 5 Mass spectrometry (MS) detection of vasopressin

Waters micromass ZQ single quadrupole electrospray ionization mass spectrometry (ESI-MS) was used to determine the purified vasopressin obtained in Examples 2, 3 and 4, where the MS analysis was carried out under the following conditions:
ion source: electron spray ion source (ESI+);
capillary ionization voltage: 3.0 kV;
cone voltage: 35 kV;
ion source temperature: 115°C;
desolvation temperature: 350°C;
desolvation nitrogen flow rate: 700 L/h;
cone counter-blow nitrogen: 50 L/h; and
scan range (*m*/*z*): 50.0-1500.

It can be seen from the detection results that a mass-to-charge ratio (*m*/*z*) of the molecular ion peak [M+H]⁺ was 1084.41, and a primary ion fragment peak [M+2H]²⁺ had a mass-to-charge ratio (*m*/*z*) of 542.71, which were consistent with the theoretical molecular weight (1084.24) of vasopressin.

## Claims

1. A method for refining vasopressin, comprising:
subjecting a crude vasopressin solution to reversed-phase enrichment, reversed-phase salt conversion and reversed-phase purification sequentially using reversed-phase high performance liquid chromatography (RP-HPLC);
wherein a packing material used in the RP-HPLC is a water-resistant packing material;
the reversed-phase enrichment, the reversed-phase salt conversion and the reversed-phase purification are all completed in one reversed-phase elution, and conditions of the reversed-phase elution are listed as follows:
| Steps | Time | Eluent |
|---|---|---|
| 1 | 0-50 min | 100% sample C1 |
| 2 | 51-71 min | 100% mobile phase C2 |
| 3 | 72-90 min | 100% mobile phase A |
| 4 | 90-95 min | 100% mobile phase A→90% mobile phase A+10% mobile phase B |
| 5 | 95-125 min | 90% mobile phase A+10% mobile phase B→80% mobile phase A+20% mobile phase B |
wherein the mobile phase A consists of 0.005-0.1% by volume of acetic acid and water; the mobile phase B consists of 0.005-0.1% by volume of acetic acid and acetonitrile; the sample C1 is the crude vasopressin solution; the mobile phase C2 is a 5-50 mM aqueous NH₄Ac-NH₄OH solution at pH 7.0-9.0; and a flow rate of the eluent is 80-100 mL/min; and
collecting an eluate within a retention time of 105-115 min to obtain a pure vasopressin solution.

2. The method according to claim 1, **characterized in that** the crude vasopressin solution is obtained by dissolving, diluting, and oxidizing a crude reduced vasopressin product prepared by solid phase synthesis.

3. The method according to claim 1, **characterized in that** the crude reduced vasopressin product is dissolved and diluted to produce a solution of the crude reduced vasopressin product; a concentration of the solution of the crude reduced vasopressin product is 0.1-4 mg/mL, preferably 0.5-2 mg/mL; and a solvent for dissolving the crude reduced vasopressin product is a 50% aqueous acetic acid solution.

4. The method according to claim 1, **characterized in that** a formula of vasopressin in the crude vasopressin solution is Cys-Tyr-Phe-Gln-Asn-Cys-Pro-Arg-Gly-NH2; and a solvent of the crude vasopressin solution is an aqueous solution containing trifluoroacetic acid and acetic acid.

5. The method according to claim 1, **characterized in that** the water-resistant packing material is UniSil® ODS-AQ material.

6. The method according to claim 1, **characterized in that** the water-resistant packing material has a pore diameter of 7-10 nm and a particle size of 10 µm.

7. The method according to claim 1, **characterized in that** a detection wavelength of the RP-HPLC is 220 nm.

8. The method according to claim 1, **characterized in that** the mobile phase A consists of 0.02-0.05% by volume of acetic acid and water; and/or
the mobile phase B consists of 0.02-0.05% by volume of acetic acid and acetonitrile; and/or
the mobile phase C2 is a 10-20 mM aqueous NH₄Ac-NH₄OH solution; and/or
the pH of the mobile phase C2 is 7.5-8.5; and/or
a HPLC purity of crude vasopressin in the crude vasopressin solution is 60-85%, preferably 70%-85%, and more preferably 70%-80%.

9. The method according to claim 1, **characterized in that** during a period of 50-51 min, the eluent is changed from the sample C1 to the mobile phase C2; and during a period of 71-72 min, the eluent is changed from the mobile phase C2 to the mobile phase A.

10. The method according to claim 1, **characterized in that** in steps (4) and (5) of the reversed-phase elution, a proportion of the mobile phase A in the eluent decreases at a uniform rate and a proportion of the mobile phase B in the eluent increases at a uniform rate.
